(19) Europäisches Patentamt / European Patent Office / Office européen des brevets

(11) **EP 2 971 036 B1**

(12) **EUROPEAN PATENT SPECIFICATION**

(45) Date of publication and mention
of the grant of the patent:
**21.02.2018 Bulletin 2018/08**

(51) Int Cl.:
*C12P 21/00* (2006.01)     *C12N 5/00* (2006.01)

(21) Application number: **14715423.1**

(86) International application number:
**PCT/IB2014/059756**

(22) Date of filing: **13.03.2014**

(87) International publication number:
**WO 2014/141151 (18.09.2014 Gazette 2014/38)**

(54) **USE OF TRICARBOXYLIC ACID (TCA) INTERMEDIATES TO CONTROL AMMONIA GENERATION IN CELL CULTURE**

VERWENDUNG VON TRICARBONSÄURE-ZWISCHENPRODUKTEN ZUR STEUERUNG DER AMMONIAK-ERZEUGUNG IN EINER ZELLKULTUR

UTILISATION D'INTERMÉDIAIRES DE L'ACIDE TRICARBOXYLIQUE (ATC) EN VUE DE LA RÉGULATION DE LA GÉNÉRATION D'AMMONIAC DANS DES CULTURES CELLULAIRES

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **15.03.2013 US 201361787105 P**

(43) Date of publication of application:
**20.01.2016 Bulletin 2016/03**

(73) Proprietor: **GlaxoSmithKline Intellectual Property (No. 2) Limited**
**Brentford, Middlesex TW8 9GS (GB)**

(72) Inventors:
• **LARA-VELASCO, Oscar**
  **King of Prussia, Pennsylvania 19406 (US)**
• **WAEHNER, Christina Michele**
  **King of Prussia, Pennsylvania 19406 (US)**

(74) Representative: **Bhogal, Jasber Kaur**
**GlaxoSmithKline**
**Global Patents (CN925.1)**
**980 Great West Road**
**Brentford, Middlesex TW8 9GS (GB)**

(56) References cited:
**WO-A2-03/106661     US-A1- 2008 254 513**

• **GENZEL YVONNE ET AL: "Substitution of glutamine by pyruvate to reduce ammonia formation and growth inhibition of mammalian cells", BIOTECHNOLOGY PROGRESS, AMERICAN INSTITUTE OF CHEMICAL ENGINEERS, US, vol. 21, no. 1, 1 January 2005 (2005-01-01), pages 58-69, XP002508714, ISSN: 8756-7938, DOI: 10.1021/BP049827D [retrieved on 2004-12-03]**
• **HASSELL T ET AL: "ADAPTATION TO NON-AMMONIAGENIC MEDIUM AND SELECTIVE SUBSTRATE FEEDING LEAD TO ENHANCED YIELDS IN ANIMAL CELL CULTURES", JOURNAL OF CELL SCIENCE, CAMBRIDGE UNIVERSITY PRESS, LONDON, GB, vol. 96, no. 3, 1 July 1990 (1990-07-01), pages 501-508, XP001079817, ISSN: 0021-9533**
• **SUTHASINEE NILSANG ET AL: "Effect of [alpha]-Ketoglutarate on Monoclonal Antibody Production of Hybridoma Cell Lines in Serum-Free and Serum-Containing Medium", APPLIED BIOCHEMISTRY AND BIOTECHNOLOGY, vol. 151, no. 2-3, 1 December 2008 (2008-12-01), pages 489-501, XP55122398, ISSN: 0273-2289, DOI: 10.1007/s12010-008-8225-0**

**Description**

Field of the Invention

The present invention relates to the field of cell culture media and methods of growing mammalian cells engineered to express therapeutic proteins.

Background of the Invention

The tricarboxylic acid (TCA) cycle is considered the essential pathway to generate energy for cells. This pathway is the final common pathway to obtain energy from fuel molecules. Sugars, amino acids and fatty acids are oxidized to obtain energy in form of ATP.

At the same time, the TCA supplies the cells with essential building blocks to produce other molecules in the cell. Intermediates for fatty acids, amino acids, purines, pyrimidines among others can be drawn off from several points of the TCA cycle during cell growth and division, and during protein synthesis.

In cases when there are not enough intermediates to replenish the TCA cycle, cells can use amino acids to replenish those intermediates. Conversion of those amino acids to intermediates requires deamidation as the first step, with ammonium ions ($NH_4^+$) being by-products of the conversion of amino acids to TCA intermediates. Depending on the demand for intermediates by cells, ammonium ions can accumulate in the culture medium to deleterious effects. Those ammonium ions can affect antibody glycosylation patterns, cell productivity, and cell growth when present at inhibitory concentrations.

In order to avoid deleterious levels of ammonium ion concentrations, currently, the aqueous cell growth medium with inhibitory levels of ammonia is simply discarded and replaced with fresh medium. This is inefficient as it requires additional equipment for sterilization and storage of fresh medium as well as the attendant replacement of costly serum in the fresh medium. What is needed therefore, is a method of removing ammonia and ammonium ions from mammalian cell cultures that dispenses with replacement of medium, and that may be utilized with the known wide variety of existing cell lines.

Summary of the Invention

In one aspect the present invention is directed to a method of reducing the ammonium ion concentration in a mammalian cell culture, comprising steps of:

> growing the cells in a cell culture medium, and
> contacting or administering an effective amount of a tricarboxylic acid ("TCA) intermediate composition to the cell culture medium, wherein the TCA intermediate composition comprises about 3mM to about 45mM malic acid and about 3mM to about 45 mM pyruvic acid.

In another aspect the present invention is directed to a method of maintaining or increasing cell productivity of cells engineered to express therapeutic proteins in a mammalian cell culture, wherein the ammonium ion generation is reduced, comprising steps of:

> growing the cells in a cell culture medium, and
> contacting or administering an effective amount of a TCA intermediate composition to the cell medium, wherein the TCA intermediate composition comprises about 3mM to about 45mM malic acid and about 3mM to about 45 mM pyruvic acid.

In another aspect the present invention is directed to a method of maintaining or increasing cell growth in a mammalian cell culture, wherein ammonium ion generation is reduced, comprising steps of:

> growing the cells in a cell culture medium, and
> contacting or administering an effective amount of a TCA intermediate composition to the cell culture medium, wherein the TCA intermediate composition comprises about 3mM to about 45mM malic acid and about 3mM to about 45 mM pyruvic acid.

In another aspect the present invention is directed to a method of reducing the influence of ammonium ion accumulation on antibody glycosylation patterns in a cell culture, wherein ammonium ion generation is reduced, comprising steps of:

growing the cells in a cell culture medium, and

contacting or administering an effective amount of a TCA intermediate composition to the cell culture medium, wherein the TCA intermediate composition comprises about 3mM to about 45mM malic acid and about 3mM to about 45 mM pyruvic acid.

In another aspect the present invention is directed to a cell culture medium comprising about 3mM to about 45mM malic acid and about 3mM to about 45 mM pyruvic acid.

Brief Description of the Drawings

Figure 1 depicts experimental design for dose response study of the TCA cycle intermediates.

Figure 2 depicts experimental design for block # 1 of the TCA cycle intermediates interaction study.

Figure 3 depicts experimental design for block # 2 of the TCA cycle intermediates interaction study

Figure 4 depicts viable cell concentration for conditions, lines show average of duplicate shake flasks

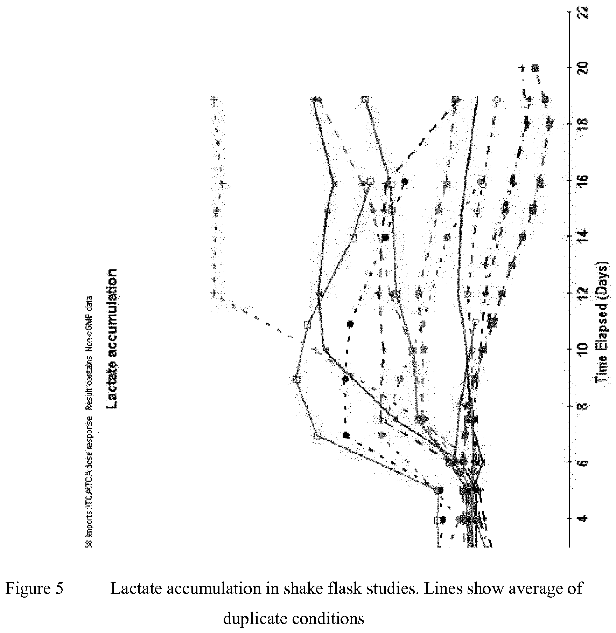

Figure 5 depicts lactate accumulation in shake flask studies. Lines show average of duplicate conditions

Figure 6 depicts ammonium accumulation for all conditions tested. Lines show average of duplicate shake flasks for each condition tested

Figure 7 depicts ammonium accumulation for $\alpha$-ketoglutarate and oxaloacetic acid addition to culture. Lines show average of duplicate shake flasks for each condition tested

Figure 8 depicts cell-specific productivity of cultures treated with different levels of TCA intermediates. Error bars show value ranges

Figure 9 depicts viable cell concentration for all conditions tested in Block 1 and Block 2 of the DOE

Figure 10 depicts ammonia accumulation profiles for all conditions tested in Block 1 and Block 2 of the DOE.

Figure 11 depicts maximum ammonia accumulation for all conditions tested in Block 1 and Block 2 of the DOE

Figure 12 depicts final cell-specific productivity for all conditions tested in Block 1 and Block 2 of the DOE

Figures 13a, 13b and 13c depict pyruvate dose response compared to control culture. Concentration of $NH_4^+$ is dependent on the dose, but the effect lasts only for a few days. $NH_4^+$ production resumes once pyruvate is consumed (Figure 12b). The order of the figures show (a) Viable cell concentration, (b) $NH_4^+$ accumulation as a function of culture time and (c) cell-specific $NH_4^+$ production. In figure (c) the slope of the curve indicates cell-specific production or consumption rates.

Figures 14a, 14b and 14c depict malate dose response compared to control culture.

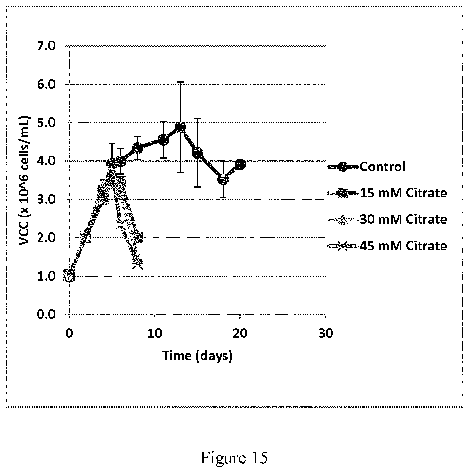

Figure 15 depicts citrate dose response compared to control culture.

Figures 16a, 16b and 16c depict malate, pyruvate, $\alpha$-ketoglutarate, fumarate, and oxaloacetate dose responses compared to control culture.

Figures 17a, 17b and 17c depict the effect of addition of two TCA intermediates to the culture.

Figures 18a, 18b and 18c depict the effect of addition of three TCA intermediates to the culture.

Figures 19 to 22 depict the average cell-specific productivity for addition of one TCA intermediate (Figures 19 and 20), combinations of two TCA intermediates (Figure 21) and three TCA intermediates (Figure 22).

Detailed Description of the Invention

Unless defined otherwise, all technical and scientific terms used herein have the same meaning as commonly understood by one of ordinary skill in the art to which the invention pertains. Any methods and materials similar or equivalent to those described herein can be used in the practice for testing of the present invention. In describing and claiming the present invention, the following terminology will be used.

It is to be understood that this invention is not limited to particular methods, reagents, compounds, compositions, or biological systems, which can, of course, vary. It is also to be understood that the terminology used herein is for the purpose of describing particular embodiments only, and is not intended to be limiting. As used in this specification and the appended claims, the singular forms "a", "an", and "the" include plural referents unless the content clearly dictates otherwise. Thus, for example, reference to "a sugar" includes a combination of two or more sugars, and the like.

"About" as used herein when referring to a measurable value such as an amount, a temporal duration, and the like, is meant to encompass variations of $\pm20\%$ or $\pm10\%$, including $\pm5\%$, $\pm1\%$, and $\pm0.1\%$ from the specified value, as such variations are appropriate to perform the disclosed methods.

In one aspect the present invention is directed to a method of reducing the ammonium ion concentration in a mammalian cell culture, comprising steps of:

growing the cells in a cell culture medium, and

contacting or administering an effective amount of a tricarboxylic acid ("TCA) intermediate composition to the cell culture medium, wherein the TCA intermediate composition comprises about 3mM to about 45mM malic acid and about 3mM to about 45 mM pyruvic acid.

In another aspect the present invention is directed to a method of maintaining or increasing cell productivity of cells engineered to express therapeutic proteins in a mammalian cell culture, wherein the ammonium ion generation is reduced, comprising steps of:

growing the cells in a cell culture medium, and

contacting or administering an effective amount of a TCA intermediate composition to the cell medium, wherein the TCA intermediate composition comprises about 3mM to about 45mM malic acid and about 3mM to about 45 mM pyruvic acid.

In another aspect the present invention is directed to a method of maintaining or increasing cell growth in a mammalian cell culture, wherein ammonium ion generation is reduced, comprising steps of:

growing the cells in a cell culture medium, and

contacting or administering an effective amount of a TCA intermediate composition to the cell culture medium, wherein the TCA intermediate composition comprises about 3mM to about 45mM malic acid and about 3mM to about 45 mM pyruvic acid.

In another aspect the present invention is directed to a method of reducing the influence of ammonium ion accumulation on antibody glycosylation patterns in a cell culture, wherein ammonium ion generation is reduced, comprising steps of:

growing the cells in a cell culture medium, and

contacting or administering an effective amount of a TCA intermediate composition to the cell culture medium, wherein the TCA intermediate composition comprises about 3mM to about 45mM malic acid and about 3mM to about 45 mM pyruvic acid.

In another aspect the present invention is directed to a cell culture medium comprising about 3mM to about 45mM malic acid and about 3mM to about 45 mM pyruvic acid.

In certain embodiments the TCA intermediates are the potassium or sodium salts of pyruvic acid and malic acid.

In one embodiment malic acid (or a salt thereof) was added to the culture to a final concentration of about 5 mM, about 10 mM, or about 15mM.

In certain embodiments pyruvic acid (or a salt thereof) was added to the culture to a final concentration of about 15 mM, about 30 mM, or about 45mM.

The TCA intermediate composition comprises about 3 mM to about 45 mM malic acid (or a salt thereof) and about 3 mM to about 45 mM pyruvic acid (or a salt thereof).

The terms "cell culture medium" and "culture medium" and "fermentation medium" refer to a nutrient solution used for growing mammalian cells that typically provides at least one component from one or more of the following categories:

1) an energy source, usually in the form of a carbohydrate such as glucose;
2) all essential amino acids, and usually the basic set of twenty amino acids plus cysteine;
3) vitamins and/or other organic compounds required at low concentrations;
4) free fatty acids; and
5) trace elements, where trace elements are defined as inorganic compounds or naturally occurring elements that are typically required at very low concentrations, usually in the micromolar range.

The nutrient solution may optionally be supplemented with one or more components from any of the following categories:

1) hormones and other growth factors as, for example, insulin, transferrin, and epidermal growth factor;
2) salts and buffers as, for example, calcium, magnesium, and phosphate;
3) nucleosides and bases such as, for example, adenosine, thymidine, and hypoxanthine; and
4) protein and tissue hydrolysates.

The cell culture medium is generally "serum free", when the medium is essentially free of serum from any mammalian source (e.g. fetal bovine serum [FBS]). By "essentially free" is meant that the cell culture medium comprises between about 0-5% serum, preferably between about 0-1% serum and most preferably between about 0-0.1% serum.

The term "mammalian host cell", "host cell", "mammalian cell" and the like, refer to cell lines derived from mammals that are capable of growth and survival when placed in either monolayer culture or in suspension culture in a medium containing the appropriate nutrients and growth factors. The necessary growth factors for a particular cell line are readily determined empirically without undue experimentation, as described for example in Mammalian Cell Culture, Mather, J. P. ed., Plenum Press, N.Y. (1984), and Barnes and Sato, (1980) Cell, 22:649. Typically, the cells are capable of expressing and secreting large quantities of a particular glycoprotein of interest into the culture medium. Examples of suitable mammalian host cells within the context of the present invention may include Chinese hamster ovary cells, CHO/-DHFR (CHO, Urlaub and Chasin, Proc. Natl. Acad. Sci. USA, 77:4216 (1980)); dp12.CHO cells (EP 307,247 published Mar. 15, 1989); mouse sertoli cells (TM4, Mather, Biol. Reprod., 23:243-251 (1980)); human cervical carcinoma cells (HELA, ATCC CCL 2); human lung cells (W138, ATCC CCL 75); human liver cells (Hep G2, HB 8065); mouse mammary tumor (MMT 060562, ATCC CCL51); TRI cells (Mather et al., Annals N.Y. Acad. Sci., 383:44-68 [1982]); MRC 5 cells; FS4 cells.

In certain embodiments, the cell-specific productivity of the cell is also maintained. In certain embodiments the cell is selected from the group consisting of selected from the group consisting of CHO cells, NS0 cells, Sp2/0 cells, COS cells, K562 cells, BHK cells, PER.C6 cells, and HEK cells. In certain embodiments the cell is a CHO cell or a subclone of CHO cells including but not limited to, CHO K1, CHO pro3-, CHO DUXB11 and CHO DG44 cells.

"Growth phase" of the cell culture refers to the period of exponential cell growth (the log phase) where cells are generally rapidly dividing. During this phase, cells are cultured for a period of time, usually between 1-5 days, and under such conditions that cell growth is maximized. The determination of the growth cycle for the host cell can be determined for the particular host cell envisioned without undue experimentation. "Period of time and under such conditions that cell growth is maximized" and the like, refer to those culture conditions that, for a particular cell line, are determine to be optimal for cell growth and divisions. During the growth phase, cells are cultured in nutrient medium containing the necessary additives generally at about 30°-40° C., preferably at about 37° C., in a humidified, controlled atmosphere, such that optimal cell growth is achieved for a particular cell line. Cells are maintained in the growth phase for a period of about between one and five days, usually between two to three days.

"Transition phase" of the cell culture refers to the period of time during which culture conditions for the production phase are engaged. During the transition phase environmental factors such as copper ion concentration and temperature are shifted from growth conditions to production conditions.

"Production phase" of the cell culture refers to the period of time during which cell growth has plateaued or is maintained at a near constant level. During the production phase, logarithmic cell growth has ended and protein production is primary. During this period of time the medium is generally supplemented to support continued protein production and to achieve the desired glycoprotein product.

The term "expression" or "expresses" are used herein to refer to transcription and translation occurring within a host cell. The level of expression of a product gene in a host cell may be determined on the basis of either the amount of corresponding mRNA that is present in the cell or the amount of the protein encoded by the product gene that is produced by the cell. For example, mRNA transcribed from a product gene is desirably quantitated by northern hybridization. Sambrook et al., Molecular Cloning: A Laboratory Manual, pp. 7.3-7.57 (Cold Spring Harbor Laboratory Press, 1989). Protein encoded by a product gene can be quantitated either by assaying for the biological activity of the protein or by employing assays that are independent of such activity, such as western blotting or radioimmunoassay using antibodies that are capable of reacting with the protein. Sambrook et al., Molecular Cloning: A Laboratory Manual, pp. 18.1-18.88 (Cold Spring Harbor Laboratory Press, 1989).

The mammalian cell culture of the present invention is prepared in a medium suitable for the particular cell; being cultured. Commercially available media such as Ham's F10 (Sigma), Minimal Essential Medium (MEM, Sigma), RPMI-1640 (Sigma), and Dulbecco's Modified Eagle's Medium (DMEM, Sigma) are exemplary nutrient solutions. In addition, any of the media described in Ham and Wallace, (1979) Meth. Enz., 58:44; Barnes and Sato, (1980) Anal. Biochem., 102:255; U.S. Pat. Nos. 4,767,704; 4,657,866; 4,927,762; 5,122,469 or 4,560,655; International Publication Nos. WO 90/03430; and WO 87/00195, may be used as culture media. Any of these media may be supplemented as necessary with hormones and/or other growth factors (such as insulin, transferrin, or epidermal growth factor), salts (such as sodium chloride, calcium, magnesium, and phosphate), buffers (such as HEPES), nucleosides (such as adenosine and thymidine), antibiotics (such as Gentamycin™ drug), trace elements (defined as inorganic compounds usually present at final concentrations in the micromolar range) lipids (such as linoleic or other fatty acids) and their suitable carriers, and glucose or an equivalent energy source. Any other necessary supplements may also be included at appropriate concentrations that would be known to those skilled in the art.

In a particular embodiment, the mammalian host cell is a CHO cell, preferably a CHO DUX (DHFR-) or subclone thereof such as CHO K1, CHO pro3-, CHO DG44, CHO DP12 cell and a suitable medium contains a basal medium component such as a DMEM/HAM F-12 based formulation (for composition of DMEM and HAM F12 media and especially serum free media, see culture media formulations in American Type Culture Collection Catalogue of Cell Lines and Hybridomas, Sixth Edition, 1988, pages 346-349) (the formulations of medium as described in U.S. Pat. No. 5,122,469 are particularly appropriate) with modified concentrations of some components such as amino acids, salts, sugar, and vitamins, and optionally containing glycine, hypoxanthine, and thymidine; recombinant human insulin, hydrolyzed peptone, such as Protease Peptone 2

and 3, Primatone HS or Primatone RL (Difco, USA; Sheffield, England), or the equivalent; a cell protective agent, such as Pluronic F68 or the equivalent Pluronic polyol; Gentamycin; and trace elements. Preferably the cell culture media is serum free.

Polypeptides may be produced by growing cells which express the desired protein under a variety of cell culture conditions. For instance, cell culture procedures for the large or small scale production of proteins are potentially useful within the context of the present invention. Procedures including, but not limited to, a fluidized bed bioreactor, hollow fiber bioreactor, roller bottle culture, or stirred tank bioreactor system may be used, in the later two systems, with or without microcarriers, and operated alternatively in a batch, fed-batch, or continuous mode.

In a one embodiment the cell culture of the present invention is performed in a stirred tank bioreactor system and a fed batch culture procedure is employed. In one embodiment the fed batch culture of the mammalian host cells and culture medium are supplied to a culturing vessel initially and additional culture nutrients are fed, continuously or in discrete increments, to the culture during culturing, with or without periodic cell and/or product harvest before termination of culture. The fed batch culture can include, for example, a semi-continuous fed batch culture, wherein periodically whole culture (including cells and medium) is removed and replaced by fresh medium. Fed batch culture is distinguished from simple batch culture in which all components for cell culturing (including the cells and all culture nutrients) are supplied to the culturing vessel at the start of the culturing process. Fed batch culture can be further distinguished from perfusion culturing insofar as the supernate is not removed from the culturing vessel during the process (in perfusion culturing, the cells are restrained in the culture by, e.g., filtration, encapsulation, anchoring to microcarriers, sedimentation, etc. and the culture medium is continuously or intermittently introduced and removed from the culturing vessel).

Further, the cells of the culture may be propagated according to any scheme or routine that may be suitable for the particular host cell and the particular production plan contemplated. Therefore, the present invention contemplates a single step or multiple step culture procedure. In a single step culture the host cells are inoculated into a culture environment and the processes of the instant invention are employed during a single production phase of the cell culture. Alternatively, a multi-stage culture is envisioned. In the multi-stage culture cells may be cultivated in a number of steps or phases. For instance, cells may be grown in a first step or growth phase culture wherein cells, possibly removed from storage, are inoculated into a medium suitable for promoting growth and high viability. The cells may be maintained in the growth phase for a suitable period of time by the addition of fresh medium to the host cell culture.

According to a another aspect of the invention, fed batch or continuous cell culture conditions are devised to enhance growth of the mammalian cells in the growth phase of the cell culture. In the growth phase cells are grown under conditions and for a period of time that is maximized for growth. Culture conditions, such as temperature, pH, dissolved oxygen ($dO_2$) and the like, are those used with the particular host and will be apparent to the ordinarily skilled artisan. Generally, the pH is adjusted to a level between about 6.5 and 7.5 using either an acid (e.g., $CO_2$) or a base (e.g., $Na_2CO_3$ or NaOH). A suitable temperature range for culturing mammalian cells such as CHO cells is between about 30 to 38° C. and preferably about 37° C. and a suitable $dO_2$ is between 5-90% of air saturation.

At a particular stage the cells may be used to inoculate a production phase or step of the cell culture. Alternatively, as described above the production phase or step may be continuous with the inoculation or growth phase or step. In one embodiment the cell culture is a fed batch cell culture.

"Polypeptide," "peptide" and "protein" are used interchangeably herein to refer to a polymer of amino acid residues. A polypeptide can be of natural (tissue-derived) origins, recombinant or natural expression from prokaryotic or eukaryotic cellular preparations, or produced chemically via synthetic methods. The terms apply to amino acid polymers in which one or more amino acid residue is an artificial chemical mimetic of a corresponding naturally occurring amino acid, as well as to naturally occurring amino acid polymers and non-naturally occurring amino acid polymer. Amino acid mimetics refers to chemical compounds that have a structure that is different from the general chemical structure of an amino acid, but that functions in a manner similar to a naturally occurring amino acid. Non-natural residues are well described in the scientific and patent literature; a few exemplary non-natural compositions useful as mimetics of natural amino acid residues and guidelines are described below. Mimetics of aromatic amino acids can be generated by replacing by, e.g., D- or L-naphylalanine; D- or L-phenylglycine; D- or L-2 thieneylalanine; D- or L-1, -2,3-, or 4-pyreneylalanine; D- or L-3 thieneylalanine; D- or L-(2-pyridinyl)-alanine; D- or L-(3-pyridinyl)-alanine; D- or L-(2-pyrazinyl)-alanine; D- or L-(4-isopropyl)-phenylglycine: D-(trifluoromethyl)-phenylglycine; D-(trifluoromethyl)-phenylalanine: D-p-fluoro-phenylalanine; D- or L-p-biphenylphenylalanine; K- or L-p-methoxy-biphenylphenylalanine: D- or L-2-indole(alkyl)alanines; and, D- or L-alkylainines, where alkyl can be substituted or unsubstituted methyl, ethyl, propyl, hexyl, butyl, pentyl, isopropyl, iso-butyl, sec-isotyl, iso-pentyl, or non-acidic amino acids. Aromatic rings of a non-natural amino acid include, e.g.,

thiazolyl, thiophenyl, pyrazolyl, benzimidazolyl, naphthyl, furanyl, pyrrolyl, and pyridyl aromatic rings.

"Peptide" as used herein includes peptides which are conservative variations of those peptides specifically exemplified herein. "Conservative variation" as used herein denotes the replacement of an amino acid residue by another, biologically similar residue. Examples of conservative variations include, but are not limited to, the substitution of one hydrophobic residue such as isoleucine, valine, leucine, alanine, cysteine, glycine, phenylalanine, proline, tryptophan, tyrosine, norleucine or methionine for another, or the substitution of one polar residue for another, such as the substitution of arginine for lysine, glutamic for aspartic acids, or glutamine for asparagine, and the like. Neutral hydrophilic amino acids which can be substituted for one another include asparagine, glutamine, serine and threonine. "Conservative variation" also includes the use of a substituted amino acid in place of an unsubstituted parent amino acid provided that antibodies raised to the substituted polypeptide also immunoreact with the unsubstituted polypeptide. Such conservative substitutions are within the definition of the classes of the peptides of the invention. "Cationic" as used herein refers to any peptide that possesses a net positive charge at pH 7.4. The biological activity of the peptides can be determined by standard methods known to those of skill in the art and described herein.

"Recombinant" when used with reference to a protein indicates that the protein has been modified by the introduction of a heterologous nucleic acid or protein or the alteration of a native nucleic acid or protein.

As used herein a "therapeutic protein" refers to any protein and/or polypeptide that can be administered to a mammal to elicit a biological or medical response of a tissue, system, animal or human that is being sought, for instance, by a researcher or clinician. A therapeutic protein may elicit more than one biological or medical response. Furthermore, the term "therapeutically effective amount" means any amount which, as compared to a corresponding subject who has not received such amount, results in, but is not limited to, healing, prevention, or amelioration of a disease, disorder, or side effect, or a decrease in the rate of advancement of a disease or disorder. The term also includes within its scope amounts effective to enhance normal physiological function as well as amounts effective to cause a physiological function in a patient which enhances or aids in the therapeutic effect of a second pharmaceutical agent.

All "amino acid" residues identified herein are in the natural L-configuration. In keeping with standard polypeptide nomenclature, abbreviations for amino acid residues are as follows:

| 1 Letter | 3 Letter | Amino Acid |
|---|---|---|
| Y | Tyr | L-tyrosine |
| G | Gly | L-glycine |
| F | Phe | L-phenylalanine |
| M | Met | L-methionine |
| A | Ala | L-alanine |
| S | Ser | L-serine |
| I | Ile | L-isoleucine |
| L | Leu | leucine |
| T | Thr | L-threonine |
| V | Val | L-valine |
| P | Pro | L-proline |
| K | Lys | L-lysine |
| H | His | L-histidine |
| Q | Gln | L-glutamine |
| E | Glu | L-glutamic acid |
| W | Trp | L-tryptophan |
| R | Arg | L-arginine |
| D | Asp | L-aspartic acid |
| N | Asn | L-asparagine |
| C | Cys | L-cysteine |

It should be noted that all amino acid residue sequences are represented herein by formulae whose left to right orientation is in the conventional direction of amino-terminus to carboxy-terminus.

In another embodiment the polypeptide is an antigen binding polypeptide. In one embodiment the antigen binding polypeptide is selected from the group consisting of a soluble receptor, antibody, antibody fragment, immunoglobulin single variable domain, Fab, F(ab')2, Fv, disulphide linked Fv, scFv, closed conformation multispecific antibody, disulphide-linked scFv, or diabody.

The term "antigen binding polypeptide" as used herein refers to antibodies, antibody fragments and other protein constructs which are capable of binding to an antigen.

The terms Fv, Fc, Fd, Fab, or F(ab)2 are used with their standard meanings (see, e.g., Harlow et al., Antibodies A Laboratory Manual, Cold Spring Harbor Laboratory, (1988)).

A "chimeric antibody" refers to a type of engineered antibody which contains a naturally-occurring variable region (light chain and heavy chains) derived from a donor antibody in association with light and heavy chain constant regions derived from an acceptor antibody.

A "humanized antibody" refers to a type of engineered antibody having its CDRs derived from a non-human donor immunoglobulin, the remaining immunoglobulin-derived parts of the molecule being derived from one (or more) human immunoglobulin(s). In addition, framework support residues may be altered to preserve binding affinity (see, e.g., Queen et al., Proc. Natl. Acad Sci USA, 86:10029-10032 (1989), Hodgson et al., Bio/Technology, 9:421 (1991)). A suitable human acceptor antibody may be one selected from a conventional database, e.g., the KABAT.RTM. database, Los Alamos database, and Swiss Protein database, by homology to the nucleotide and amino acid sequences of the donor antibody. A human antibody characterized by a homology to the framework regions of the donor antibody (on an amino acid basis) may be suitable to provide a heavy chain constant region and/or a heavy chain variable framework region for insertion of the donor CDRs. A suitable acceptor antibody capable of donating light chain constant or variable framework regions may be selected in a similar manner. It should be noted that the acceptor antibody heavy and light chains are not required to originate from the same acceptor antibody. The prior art describes several ways of producing such humanized antibodies--see for example EP-A-0239400 and EP-A-054951.

The term "donor antibody" refers to an antibody (monoclonal, and/or recombinant) which contributes the amino acid sequences of its variable regions, CDRs, or other functional fragments or analogs thereof to a first immunoglobulin partner, so as to provide the altered immunoglobulin coding region and resulting expressed altered antibody with the antigenic specificity and neutralizing activity characteristic of the donor antibody.

The term "acceptor antibody" refers to an antibody (monoclonal and/or recombinant) heterologous to the donor antibody, which contributes all (or any portion, but in some embodiments all) of the amino acid sequences encoding its heavy and/or light chain framework regions and/or its heavy and/or light chain constant regions to the first immunoglobulin partner. In certain embodiments a human antibody is the acceptor antibody.

"CDRs" are defined as the complementarity determining region amino acid sequences of an antibody which are the hypervariable regions of immunoglobulin heavy and light chains. See, e.g., Kabat et al., Sequences of Proteins of Immunological Interest, 4th Ed., U.S. Department of Health and Human Services, National Institutes of Health (1987). There are three heavy chain and three light chain CDRs (or CDR regions) in the variable portion of an immunoglobulin. Thus, "CDRs" as used herein refers to all three heavy chain CDRs, or all three light chain CDRs (or both all heavy and all light chain CDRs, if appropriate). The structure and protein folding of the antibody may mean that other residues are considered part of the antigen binding region and would be understood to be so by a skilled person. See for example Chothia et al., (1989) Conformations of immunoglobulin hypervariable regions; Nature 342, p 877-883.

As used herein the term "domain" refers to a folded protein structure which has tertiary structure independent of the rest of the protein. Generally, domains are responsible for discrete functional properties of proteins and in many cases may be added, removed or transferred to other proteins without loss of function of the remainder of the protein and/or of the domain. An "antibody single variable domain" is a folded polypeptide domain comprising sequences characteristic of antibody variable domains. It therefore includes complete antibody variable domains and modified variable domains, for example, in which one or more loops have been replaced by sequences which are not characteristic of antibody variable domains, or antibody variable domains which have been truncated or comprise N- or C-terminal extensions, as well as folded fragments of variable domains which retain at least the binding activity and specificity of the full-length domain.

The phrase "immunoglobulin single variable domain" refers to an antibody variable domain ($V_H$, $V_{HH}$, $V_L$) that specifically binds an antigen or epitope independently of a different V region or domain. An immunoglobulin single variable domain can be present in a format (e.g., homo- or hetero-multimer) with other, different variable regions or variable domains where the other regions or domains are not required for antigen binding by the single immunoglobulin variable domain (i.e., where the immunoglobulin single variable domain binds antigen independently of the additional variable domains). A "domain antibody" or "dAb" is the same as an "immunoglobulin single variable domain" which is capable of binding to an antigen as the term is used herein. An immunoglobulin single variable domain may be a human antibody variable domain, but also includes single antibody variable domains from other species such as rodent (for example, as disclosed in WO 00/29004), nurse shark and Camelid $V_{HH}$ dAbs (nanobodies). Camelid $V_{HH}$ are immunoglobulin single variable domain polypeptides that are derived from species including camel, llama, alpaca, dromedary, and guanaco, which produce heavy chain antibodies naturally devoid of light chains. Such $V_{HH}$ domains may be humanized according to standard techniques available in the art, and such domains are still considered to be "domain antibodies" according to the invention. As used herein "$V_H$ includes camelid $V_{HH}$ domains. NARV are another type of immunoglobulin single variable domain which were identified in cartilaginous fish including the nurse shark. These domains are also known as Novel Antigen Receptor variable region (commonly abbreviated to V(NAR) or NARV). For further details see Mol. Immunol. 44, 656-665 (2006) and US20050043519A.

The term "Epitope-binding domain" refers to a domain that specifically binds an antigen or epitope independently of a different V region or domain, this may be a domain antibody (dAb), for example a human, camelid or shark immunoglobulin single variable domain.

As used herein, the term "antigen-binding site" refers to a site on a protein which is capable of specifically binding to antigen, this may be a single domain, for example an epitope-binding domain, or it may be paired $V_H$/$V_L$ domains as can be found on a standard antibody. In some aspects of the invention single-chain Fv (ScFv) domains can provide antigen-binding sites.

The terms "mAbdAb" and dAbmAb" are used herein to refer to antigen-binding proteins of the present invention. The two terms can be used interchangeably, and are intended to have the same meaning as used herein.

Example 1--Materials and methods/Experimental Design

Several TCA cycle intermediates were selected for a dose response experiment based on commercial availability, either the acid or its sodium salt were purchased from Sigma Aldrich to prepare stock solution feeds to be used in the study. Selected compounds were pyruvic acid, citric acid, α-ketoglutaric acid, fumaric acid, malic acid (or sodium salts), and oxaloacetic acid (no sodium salt available).

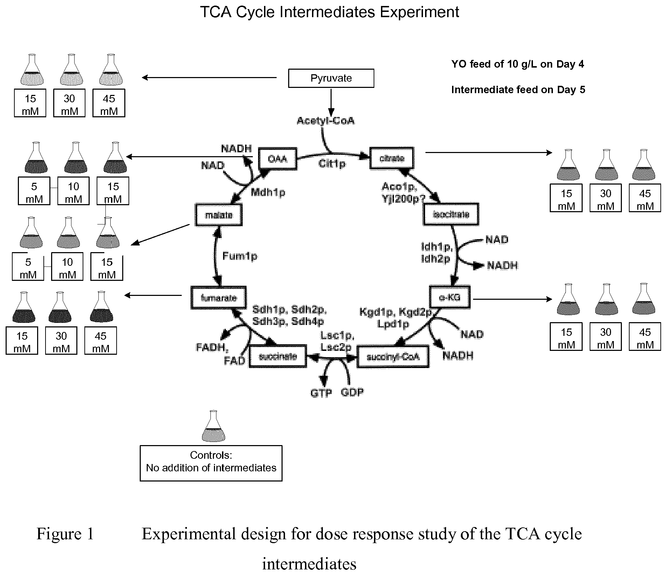

Oxaloacetic acid and malic acid were added to the culture to a final concentration of 5 mM, 10 mM and 15mM. The rest of the TCA intermediates tested were added to the culture to a final concentration of 15 mM, 30 mM and 45 mM. Figure 1 summarizes the TCA cycle components tested and their location in the pathway. Table 1 summarizes the operation condition of shake flasks in the study as well as feed schedules for the dose response study.

Control shake flasks were set up every time an arm of the study started. Those control shake flasks were fed-batch cultures without addition of any TCA intermediate. All conditions tested were executed in duplicate shake flasks.

Table 1 Operation Conditions for shake flasks for dose response study

| Cell line | DG44 CHO |
|---|---|
| Vessel volume/Working volume (mL) | 250/150 |
| Agitation speed (RPM) | 150 |
| Temperature (°C) | 35 |
| $CO_2$ concentration in incubator (%) | 5 |
| Target Initial Viable Cell Concentration (iVCC) (x $10^6$cells/mL) | 0.8-1.0 |
| Medium type | 9-01-3-0174 |
| Hydrolysate feed | On Day 4 after inoculation |
| TCA intermediate feed | On Day 5 after inoculation |

Example 2--Design of Experiment (DOE) to test interactions on selected TCA intermediates/Analytical Methods

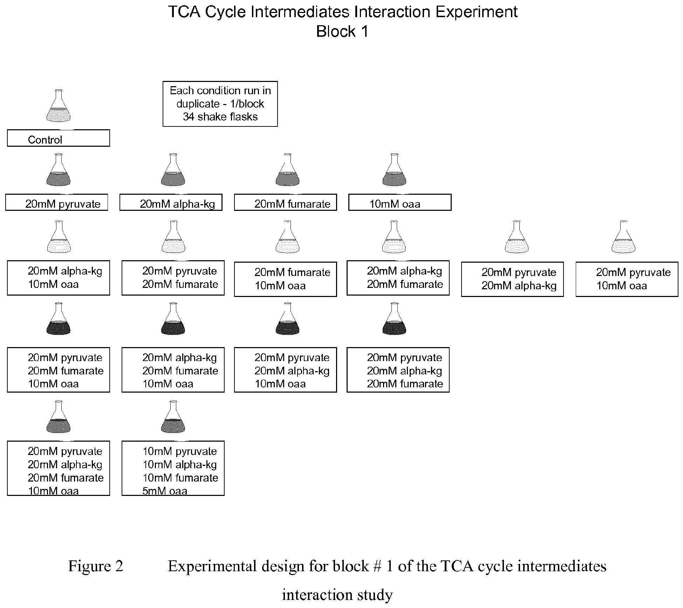

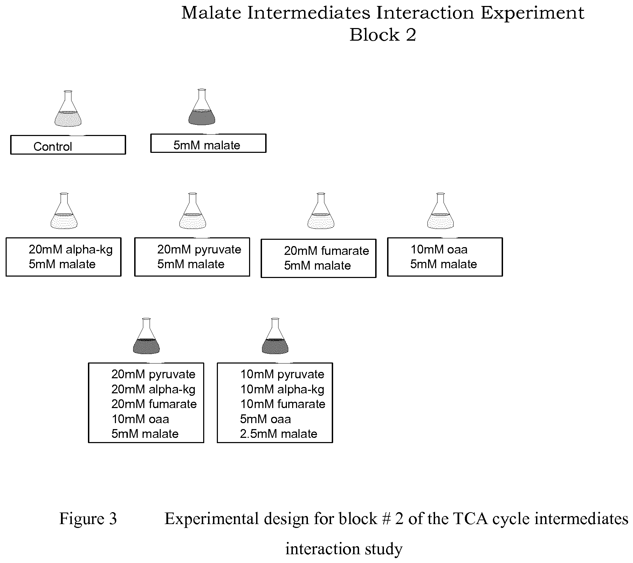

The experiment to test interactions of all the selected components was carried out in two blocks. Each one of the blocks was designed to contain center points and control shake flasks (batch cultures, no addition of any component). The first block tested interactions of pyruvic acid, α-ketoglutaric acid, fumaric acid and oxaloacetic acid. The second block tested interactions of pyruvic acid, α-ketoglutaric acid, fumaric acid, oxaloacetic acid and malic acid. Figure 2 shows the experimental set up for block# 1, and Figure 3 shows the experimental set up for block# 2. All conditions were run in duplicates. Operation conditions of shake flasks is shown in Table 2

Table 2 Operation Conditions for shake flasks for DOE study

| Cell line | DG44 CHO |
|---|---|
| Vessel volume/Working volume (mL) | 250/150 |
| Agitation speed (RPM) | 150 |
| Temperature (°C) | 35 |
| $CO_2$ concentration in incubator (%) | 5 |

(continued)

| Target Initial Viable Cell Concentration (iVCC) (x $10^6$cells/mL) | 1.0 |
|---|---|
| Medium type | 9-01-3-0174 |
| Hydrolysate feed and vitamin feed | On Day 4 after inoculation |
| TCA intermediate feed | On Day 5 after inoculation |

Shake flask cultures were sampled every other day to determine Total Cell Concentration (TCC), Viable Cell Concentration (VCC), cell viability, metabolite (glucose, lactate, glutamine, glutamate, and ammonia) concentration, and osmolality. The master sample was divided into cell-based assays (TCC, viability assays) and cell-free assays (metabolite concentration, osmolality). Back-up samples (cell-free supernatants) for future reference were produced by centrifuging a portion of the master sample and aliquoting 1.8 mL in Nunc cryovials which were subsequently stored at -70°C. Titer was determined on samples filtered through a 0.22 μm-filter.

TCC, VCC and viability were determined using an automated cell counter based on the trypan blue exclusion method (ViCell XR, Beckman Coulter). Samples were pretreated with protease prior to cell counting (TripLE, Life Technologies, USA) using equal volumes of protease stock solution and culture. Incubation to allow dissociation was carried out for 20-25 minutes at 37°C under gentle shaking. Metabolites were measured using Nova Bioprofile (Nova Bioprofile 400); which operates on enzyme-based electrochemical reactions. Osmolality measurement was based on the freezing-point depression method (Advanced Instruments, Model N/A). Filtered cell-free samples were sent to the Bioanalytical Development group for antibody accumulation and binding activity determination (Biacore).

Parameter calculation

Integral of Viable Cell Concentration (IVC)

Integral of Viable Cells was calculated using VCC data and applying the trapezoid rule following the formula,

$$IVC = \sum_{n=1}^{i}\left[ \frac{(VCC_n + VCC_{n-1})}{2}(t_n - t_{n-1}) \right]$$

(2)

Where:

IVC, Integral of Viable Cell density [=] (106 cells/mL).day
t, culture time [=]days
VCC, Viable cell density [=] 106 cells/mL
ni, number of samples taken throughout the culture, for calculation purposes i=4

Specific Productivity was calculated using the last titer data point divided by last calculated IVC value.

Example 3--Dose response results

The experiment was divided into three blocks. The first block tested pyruvic acid, citric acid, α-ketoglutaric acid and fumaric acid. The second block tested oxaloacetic acid and the third block tested malic acid.

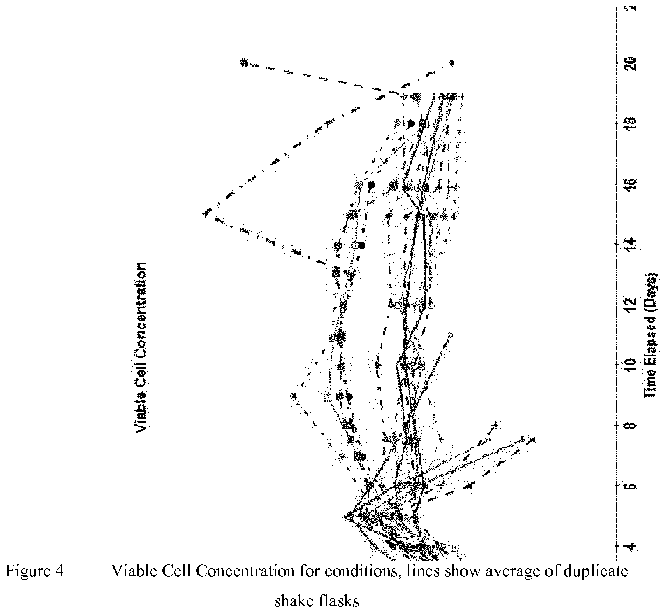

Figure 4 shows consolidated average VCC results for all shake flasks. All cultures treated with citric acid sharply lost viability within two days after addition of citric acid to the culture as indicated by the reduction in viable cell concentration. Malic acid added in high concentration (15 mM and 30 mM) to the culture also induced loss of cell viability of the culture as seen in Figure 4. In this case, addition of malic acid showed a dose response effect with the fastest decline in viability induced by the 30 mM concentration of malic acid, followed by 15mM and no decline in viability (as compared to control shake flasks) when malic acid concentration in the culture was 5 mM.

Lactate accumulation depended on the type and amount of TCA intermediate added to the culture. Control shake flasks showed low lactate accumulation (< 1.0 g/L) peaking at around day 7 followed by a lactate consumption stage.

When 45 mM pyruvic acid was added to the culture, lactate accumulation reached around 3 g/L. All other TCA intermediates influenced lactate accumulation to levels between 1 g/L to 2 g/L. Lactate accumulation profile in shake flasks added with malic acid to a final concentration of 5mM was similar to that seen in control shake flasks.

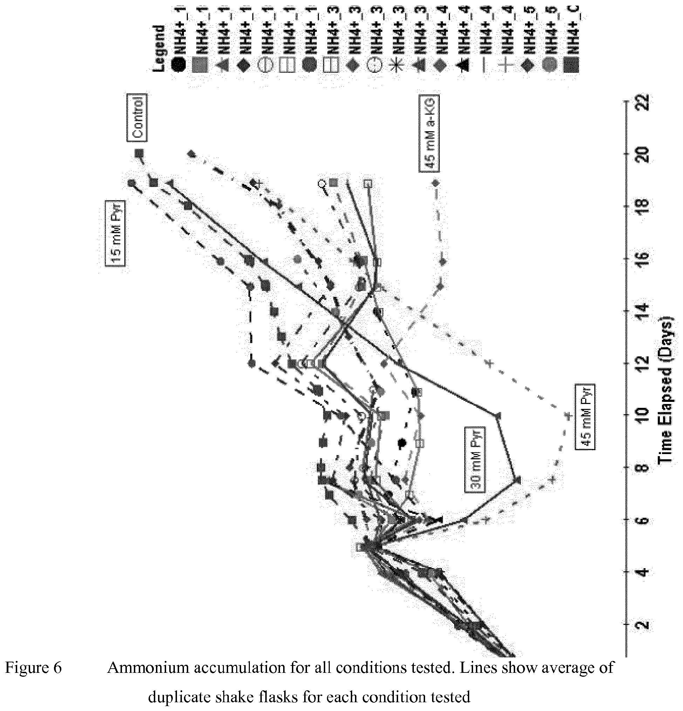

Ammonium profiles showed different levels accumulation. Ammonia concentration in shake flasks testing control conditions reached close to 9mM. Figure 6 summarizes data for all conditions tested in the dose response study. Addition of pyruvic acid to the culture to a final concentration of 45 mM on Day 5 induced ammonia consumption up to Day 10. After this time point in the culture, ammonia accumulation resumed and reached a final concentration of 7 mM. When pyruvic acid was added to 30 mM the ammonia consumption effect lasted only to Day 8 and then ammonia accumulated to 8 mM. Pyruvic acid addition to a final concentration of 15 mM only had a minor effect on the reduction of ammonia and cultures accumulated ammonia to a slightly higher level than that found in control shake flasks.

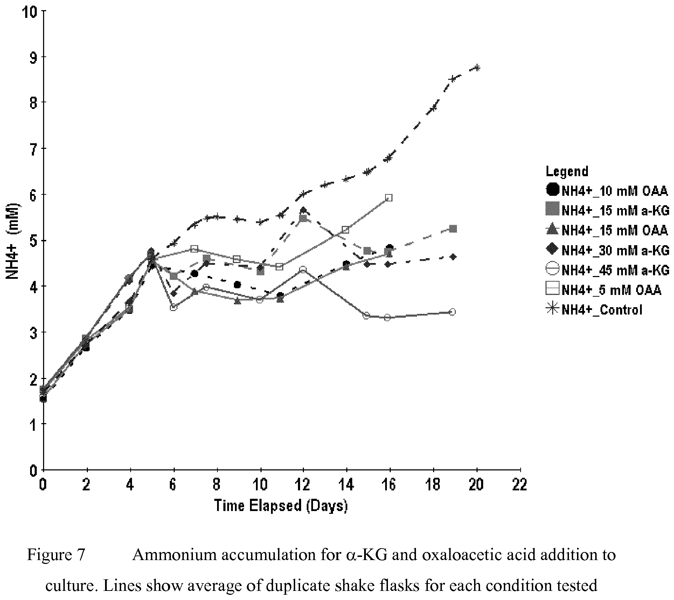

TCA intermediates that offered the best control in ammonia accumulation were $\alpha$-ketoglutaric acid and oxaloacetic acid. Those intermediates at concentrations tested maintained ammonia concentration between 3 mM and 6 mM. Figure 7 summarizes the effect of $\alpha$-ketoglutaric acid and oxaloacetic acid on ammonia accumulation.

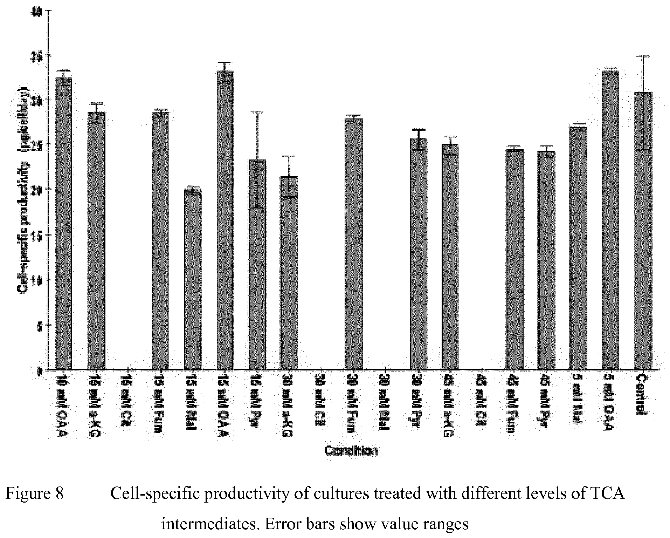

Addition of TCA intermediates to the culture also affected the final cell specific productivity. In case of control cultures, the calculated cell specific productivity value averaged 31 pg/cell/day (range 24-35 pcd); cultures treated with 45 mM of pyruvic acid showed a decrease in cell-specific productivity to ~24 pg/cell/day. Only cultures treated with oxoaloacetic acid showed a reduction in the ammonia accumulation levels and sustained cell-specific productivity values as shown in Figure 8. Addition of all other TCA intermediates showed a decrease in cell specific productivity.

Therefore, the best TCA intermediate to control ammonia and maintain cell-specific productivity levels is oxaloacetic acid in concentrations from 5-15 mM in the culture.

Example 4--Interactions among TCA intermediates (two to five). DOE results

From the dose response experiments, pyruvic acid, malic acid, oxaloacetic acid, fumaric acid, and $\alpha$-ketoglutaric acid were advanced to test interactions. Concentration of each one of the intermediates was chosen as the concentration that showed a response during the dose response experiment. In some cases, the concentration chosen was in between two values actually tested during the dose response experiment.

The experiment was set up in two blocks due to the number of shake flasks in the entire study. Figure 2 and figure 3 show the conditions tested per block. Each block contained control conditions (without addition of TCA intermediates).

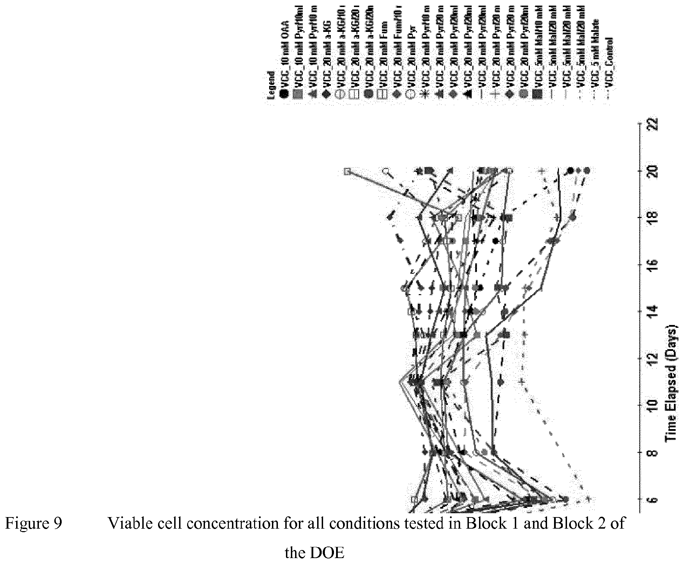

Viable Cell Concentration showed similar peak values for all conditions tested as shown in Figure 9. The drop in VCC observed on Day 6 is explained by the addition of the TCA intermediates as some dilution of the culture happened to obtain the desired final concentrations in the culture.

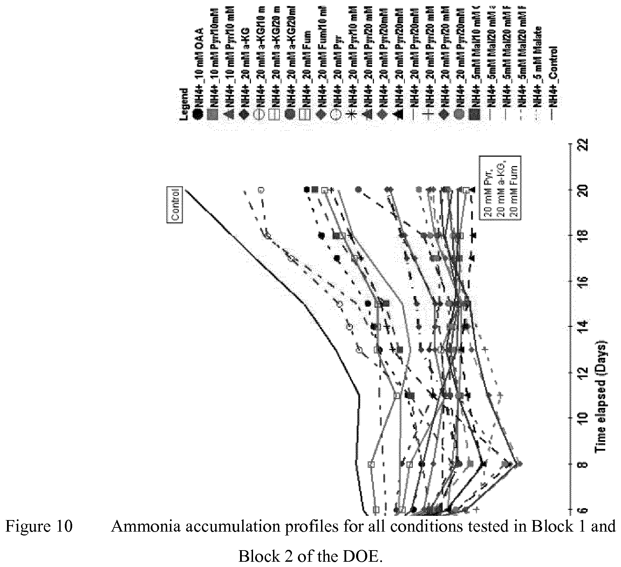

In case of ammonia accumulation, response to intermediates showed a large variation. Cultures in control conditions showed ammonia accumulation as high as 9 mM while the mixture of 20 mM pyruvic acid, 20 mM $\alpha$-ketoglutarate, 20 mM fumaric acid showed the lowest ammonia accumulation to 2 mM at the end of the culture. As observed in the dose response studies, malic acid and pyruvic acid could suppress ammonia generation for a few days and then ammonia levels rise to a final concentration similar to that of the control cultures. Figure 10 summarizes data for all conditions.

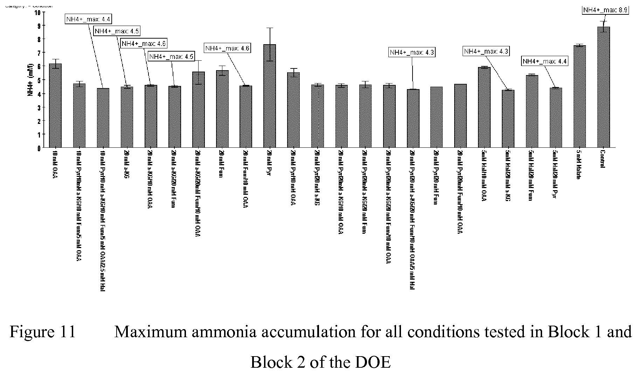

Figure 11 shows peak ammonia accumulation for all conditions tested. Text boxes show conditions with the lowest ammonia accumulation. Several of the combinations showed control over ammonia concentration. All marked conditions showed a maximum ammonia concentration close to 4 mM (Ammonia concentration in control is ~ 9 mM).

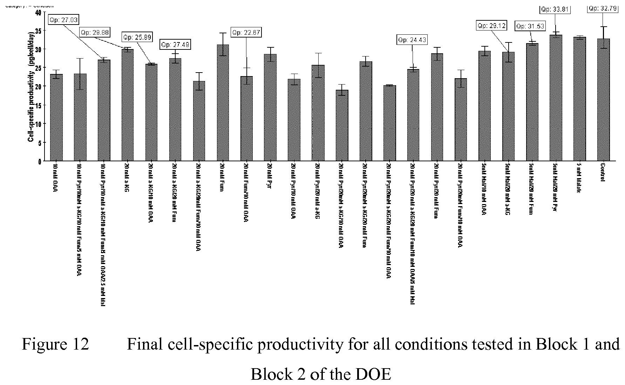

When the conditions leading to low ammonia accumulation are mapped into the cell-specific productivity data (Figure 12), results show that some of the TCA intermediate combinations have a negative effect on cell-specific productivity compared to the control.

Out of all the conditions tested, the combinations 5 mM malic acid / 20 mM 20 pyruvic acid, 5 mM malic acid / 20 mM fumaric acid, 5 mM malic acid / 20 mM $\alpha$-ketoglutaric acid, and 20 mM $\alpha$-ketoglutaric acid maintain cell-specific productivity and control ammonia generation. In all cases, cell-specific productivity was maintained at a level close to 30 pg/cell/day.

Example 5--Further analysis of TCA intermediate experiments.

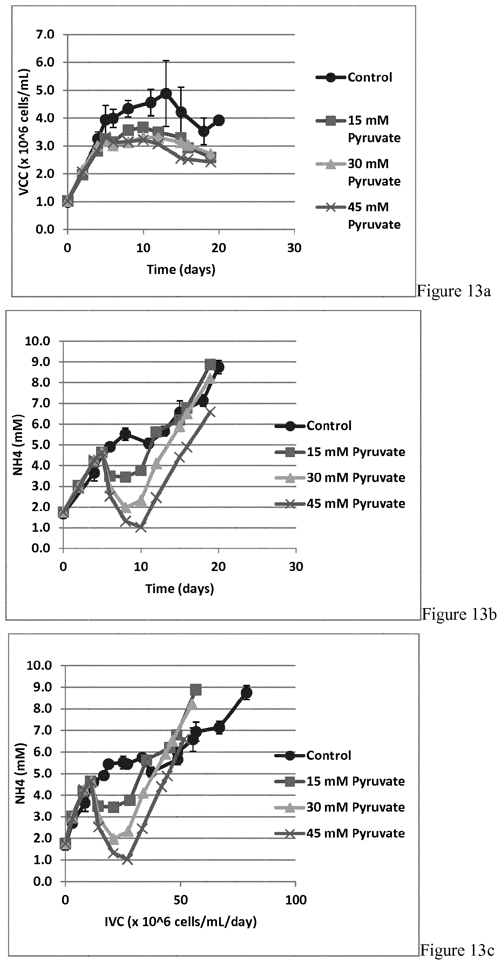

Figures 13a, 13b and 13c show pyruvate dose response compared to control culture. Concentration of $NH_4^+$ is dependent on the dose, but the effect lasts only for a few days. $NH_4^+$ production resumes once pyruvate is consumed (Figure 13b). The order of the figures show (a) Viable cell concentration, (b) $NH_4^+$ accumulation as a function of culture time and (c) cell-specific $NH_4^+$ production. In figure (c) the slope of the curve indicates cell-specific production or consumption rates.

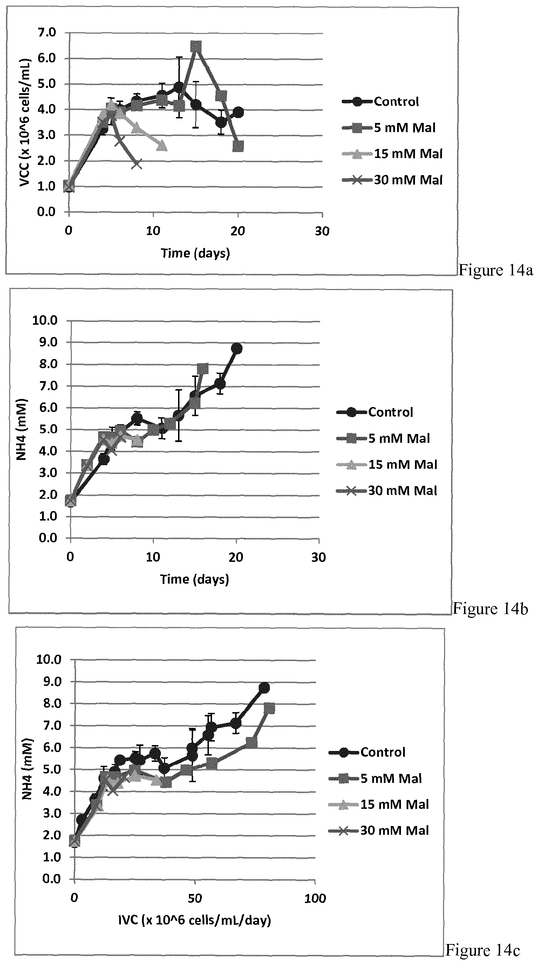

In the case of malate, the dose response experiment indicates that concentrations higher than 5 mM are toxic to cells. At 5mM concentration, there is not a clear effect of malic acid on $NH_4^+$ accumulation or production as shown in Figures 14b and 14c.

Addition of citrate had deleterious effects on culture. Its use to reduce $NH_4^+$ accumulation should be avoided.

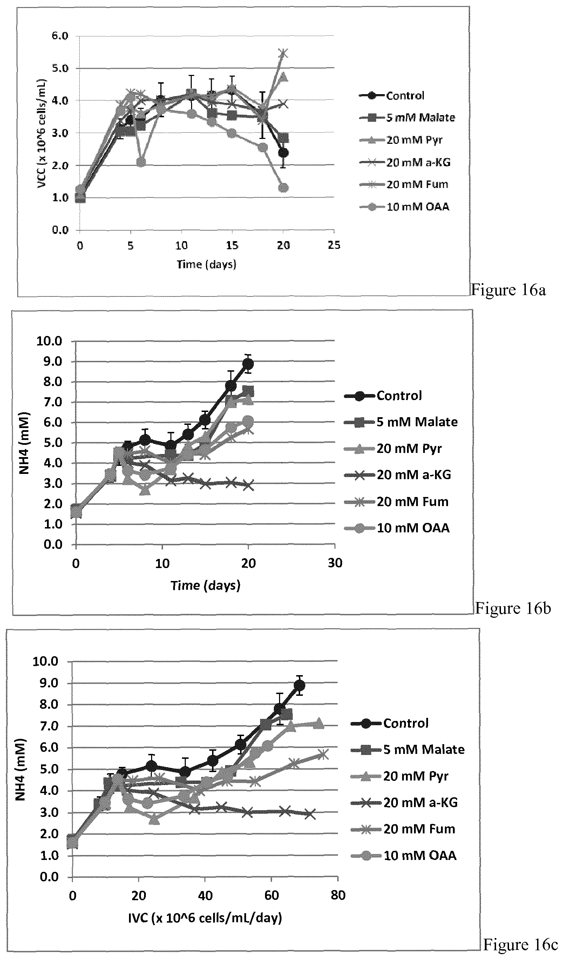

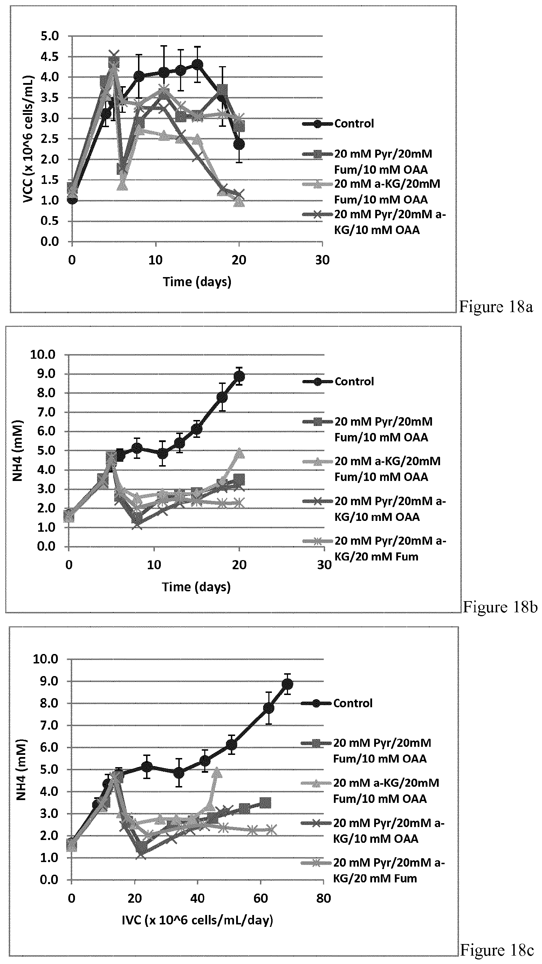

A second experiment was set up to verify results from the first experiment using selected intermediates added at a certain concentration. In this experiment, only minimal effects on cell concentration were observed while results were confirmed. Addition of malate, oxaloacetate and pyruvate partially reduced $NH_4^+$ accumulation and reduction (Figure 16b and 16c); fumarate addition slows down $NH_4^+$ production and accumulation. In case of α-KG, Figure 16c indicates that $NH_4^+$ is consumed, which is also shown in Figure 18b.

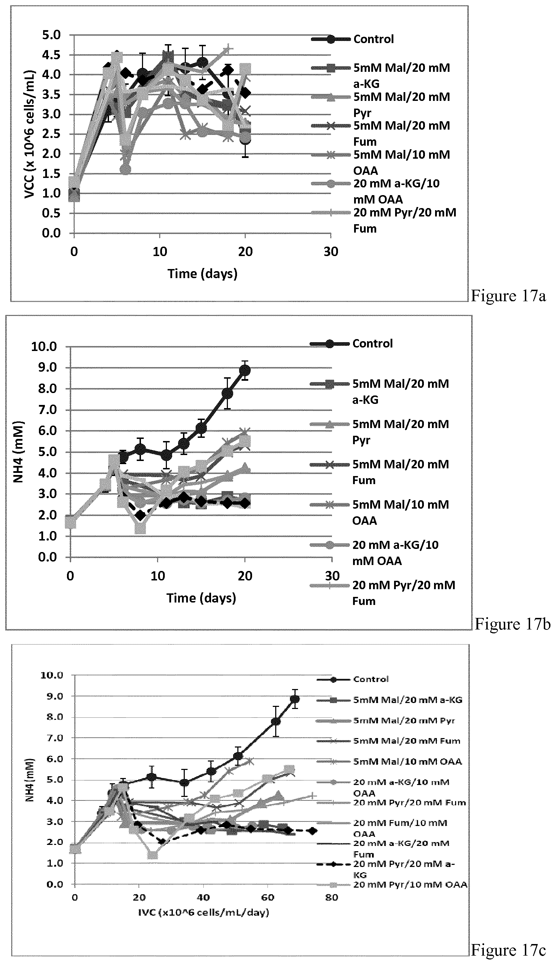

Figures 17a to 17c show the effect of addition of two TCA intermediates to the culture. In general, VCC was not greatly affected (Figure 17a) since cell counts did not show major differences compared to the control culture or recovered as culture continued.

In the case of $NH_4^+$ production and accumulation, it was evident that the addition of two TCA intermediates results in better $NH_4^+$ control. From Figure 17c, it is possible to note that combinations containing α-KG resulted in lower cell-specific production rates and stable $NH_4^+$ concentration in the culture (curves around black dotted line).

Figures 18a to 18c show the effect of addition of 3 TCA intermediates to the culture. For the combinations α-KG/Fumarate/OAA and Pyruvate/α-KG/OAA, cell counts

were significantly affected after addition of this combination suggesting that use should be avoided. In case of the combination pyruvate/α-KG/fumarate, the effect on cell counts were less pronounced. This same combination showed reduction in $NH_4^+$ production rate and stabilization of $NH_4^+$ concentration (Figures 18c and 18b, respectively).

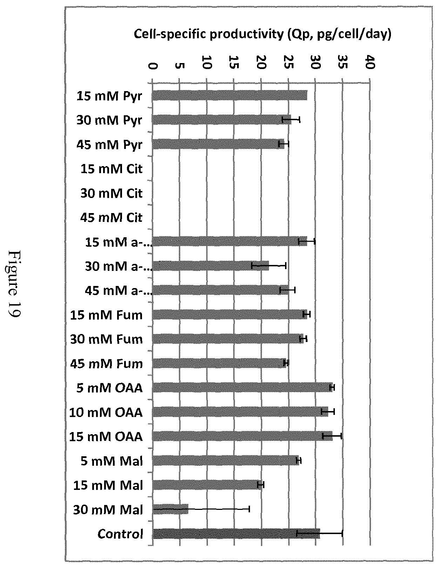

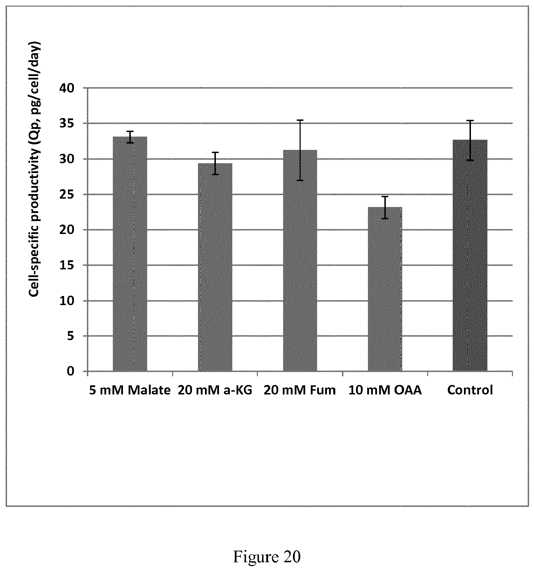

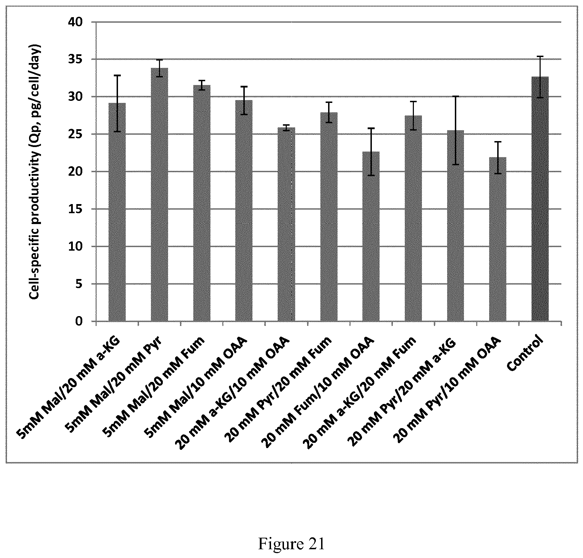

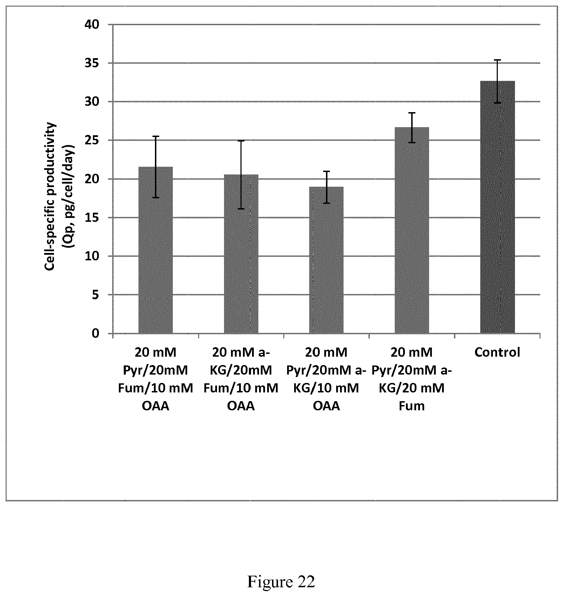

Figures 19 to 22 show the average cell-specific productivity for addition of one TCA intermediate (Figures 19 and 20), combinations of two TCA intermediates (Figure 21) and three TCA intermediates (Figure 22).

α-KG was identified as the most effective TCA intermediate to reduce $NH_4^+$ accumulation when used alone. However, it also tends to show reduction of the cell-specific productivity as shown in Figures 19 and 20 (by about 10-20% compared to the control).

In case of combinations of TCA intermediates, the combination Pyruvate/Malate (5mM/20mM) preserved cell-specific productivity while achieving acceptable $NH_4^+$ reduction (Figures 17b and 17c). There are a number of combinations possible that could be used to reduce $NH_4^+$ accumulation and they should be assessed based on the purpose. Combinations including α-KG are expected to work better than other combinations at the expense of reduced cell-specific productivity.

Finally, some combinations including 3 TCA intermediates also reduce $NH_4^+$ accumulation.

**Claims**

**1.** A method of reducing the ammonium ion concentration in a mammalian cell culture, comprising steps of:

   growing the cells in a cell culture medium, and
   contacting or administering an effective amount of a tricarboxylic acid ("TCA") intermediate composition to the cell culture medium, wherein the TCA intermediate composition comprises about 3 mM to about 45 mM malic acid and about 3 mM to about 45 mM pyruvic acid.

**2.** A method of maintaining or increasing cell productivity of cells engineered to express therapeutic proteins in a mammalian cell culture, wherein the ammonium ion generation is reduced, comprising steps of:

   growing the cells in a cell culture medium, and
   contacting or administering an effective amount of a TCA intermediate composition to the cell medium, wherein the TCA intermediate composition comprises about 3 mM to about 45 mM malic acid and about 3 mM to about 45 mM pyruvic acid.

**3.** A method of maintaining or increasing cell growth in a mammalian cell culture, wherein ammonium ion generation is reduced, comprising steps of:

   growing the cells in a cell culture medium, and
   contacting or administering an effective amount of a TCA intermediate composition to the cell culture medium, wherein the TCA intermediate composition comprises about 3 mM to about 45 mM malic acid and about 3 mM to about 45 mM pyruvic acid.

**4.** A method of reducing the influence of ammonium ion accumulation on antibody glycosylation patterns in a cell culture, wherein ammonium ion generation is reduced, comprising steps of:

growing the cells in a cell culture medium, and
contacting or administering an effective amount of a TCA intermediate composition to the cell culture medium, wherein the TCA intermediate composition comprises about 3 mM to about 45 mM malic acid and about 3 mM to about 45 mM pyruvic acid.

5. A method according to any of claims 1-4, in which cell-specific productivity is also maintained.

6. A cell culture medium comprising about 3 mM to about 45 mM malic acid and about 3 mM to about 45 mM pyruvic acid.

7. A cell culture medium according to claim 6, in which cell-specific productivity is also maintained.

**Patentansprüche**

1. Verfahren zum Reduzieren der Ammoniumionenkonzentration in einer Säugetierzellkultur, welches die folgenden Schritte umfasst:

Heranziehen der Zellen in einem Zellkulturmedium und
Inkontaktbringen oder Verabreichen einer wirksamen Menge einer Tricarbonsäure (tricarboxylic acid; TCA)-Intermediat-Zusammensetzung mit dem/in das Zellkulturmedium, wobei die TCA-Intermediat-Zusammensetzung etwa 3 mM bis etwa 45 mM Äpfelsäure und etwa 3 mM bis etwa 45 mM Pyruvinsäure umfasst.

2. Verfahren zum Aufrechterhalten oder Steigern der Zellproduktivität von Zellen, die so manipuliert wurden, dass sie therapeutische Proteine in einer Säugetierzellkultur exprimieren, wobei die Ammoniumionenerzeugung reduziert ist, welches die folgenden Schritte umfasst:

Heranziehen der Zellen in einem Zellkulturmedium und
Inkontaktbringen oder Verabreichen einer wirksamen Menge einer TCA-Intermediat-Zusammensetzung mit dem/in das Zellmedium, wobei die TCA-Intermediat-Zusammensetzung etwa 3 mM bis etwa 45 mM Äpfelsäure und etwa 3 mM bis etwa 45 mM Pyruvinsäure umfasst.

3. Verfahren zum Aufrechterhalten oder Steigern des Zellwachstums in einer Säugetierzellkultur, wobei die Ammoniumionenerzeugung reduziert ist, welches die folgenden Schritte umfasst:

Heranziehen der Zellen in einem Zellkulturmedium und
Inkontaktbringen oder Verabreichen einer wirksamen Menge einer TCA-Intermediat-Zusammensetzung mit dem/in das Zellkulturmedium, wobei die TCA-Intermediat-Zusammensetzung etwa 3 mM bis etwa 45 mM Äpfelsäure und etwa 3 mM bis etwa 45 mM Pyruvinsäure umfasst.

4. Verfahren zum Reduzieren des Einflusses der Ammoniumionenakkumulation auf Antikörperglycosylierungsmuster in einer Zellkultur, wobei die Ammoniumionenerzeugung reduziert ist, welches die folgenden Schritte umfasst:

Heranziehen der Zellen in einem Zellkulturmedium und
Inkontaktbringen oder Verabreichen einer wirksamen Menge einer TCA-Intermediat-Zusammensetzung mit dem/in das Zellkulturmedium, wobei die TCA-Intermediat-Zusammensetzung etwa 3 mM bis etwa 45 mM Äpfelsäure und etwa 3 mM bis etwa 45 mM Pyruvinsäure umfasst.

5. Verfahren gemäß irgendeinem der Ansprüche 1-4, wobei die zellspezifische Produktivität ebenfalls aufrechterhalten wird.

6. Zellkulturmedium, welches etwa 3 mM bis etwa 45 mM Äpfelsäure und etwa 3 mM bis etwa 45 mM Pyruvinsäure umfasst.

7. Zellkulturmedium gemäß Anspruch 6, wobei die zellspezifische Produktivität ebenfalls aufrechterhalten wird.

**Revendications**

1. Procédé de réduction de la concentration des ions ammonium dans une culture de cellules de mammifère, comprenant les étapes suivantes :

   la croissance des cellules dans un milieu de culture cellulaire, et
   la mise en contact ou l'administration d'une quantité efficace d'une composition d'intermédiaires d'acide tricarboxylique (ATC) au milieu de culture cellulaire, dans lequel la composition d'intermédiaires d'ATC comprend environ 3 mM à environ 45 mM d'acide malique et environ 3 mM à environ 45 mM d'acide pyruvique.

2. Procédé de maintien ou d'augmentation de la productivité cellulaire des cellules modifiées pour exprimer des protéines thérapeutiques dans une culture de cellules de mammifère, dans lequel la production des ions ammonium est réduite, comprenant les étapes suivantes :

   la croissance des cellules dans un milieu de culture cellulaire, et
   la mise en contact ou l'administration d'une quantité efficace d'une composition d'intermédiaires d'ATC au milieu cellulaire, dans lequel la composition d'intermédiaires d'ATC comprend environ 3 mM à environ 45 mM d'acide malique et environ 3 mM à environ 45 mM d'acide pyruvique.

3. Procédé de maintien ou d'augmentation de la croissance cellulaire dans une culture de cellules de mammifère, dans lequel la production des ions ammonium est réduite, comprenant les étapes suivantes :

   la croissance des cellules dans un milieu de culture cellulaire, et
   la mise en contact ou l'administration d'une quantité efficace d'une composition d'intermédiaires d'ATC au milieu de culture cellulaire, dans lequel la composition d'intermédiaires d'ATC comprend environ 3 mM à environ 45 mM d'acide malique et environ 3 mM à environ 45 mM d'acide pyruvique.

4. Procédé de réduction de l'influence de l'accumulation des ions ammonium sur les profils de glycosylation des anticorps dans une culture cellulaire, dans lequel la production des ions ammonium est réduite, comprenant les étapes suivantes :

   la croissance des cellules dans un milieu de culture cellulaire, et
   la mise en contact ou l'administration d'une quantité efficace d'une composition d'intermédiaires d'ATC au milieu de culture cellulaire, dans lequel la composition d'intermédiaires d'ATC comprend environ 3 mM à environ 45 mM d'acide malique et environ 3 mM à environ 45 mM d'acide pyruvique.

5. Procédé selon l'une quelconque des revendications 1 à 4, dans lequel la productivité spécifique des cellules est également maintenue.

6. Milieu de culture cellulaire comprenant environ 3 mM à environ 45 mM d'acide malique et environ 3 mM à environ 45 mM d'acide pyruvique.

7. Milieu de culture cellulaire selon la revendication 6, dans lequel la productivité spécifique des cellules est également maintenue.

TCA Cycle Intermediates Experiment

Figure 1        Experimental design for dose response study of the TCA cycle
intermediates

TCA Cycle Intermediates Interaction Experiment
Block 1

Each condition run in
duplicate - 1/block
34 shake flasks

Control

| 20mM pyruvate | 20mM alpha-kg | 20mM fumarate | 10mM oaa |

| 20mM alpha-kg 10mM oaa | 20mM pyruvate 20mM fumarate | 20mM fumarate 10mM oaa | 20mM alpha-kg 20mM fumarate | 20mM pyruvate 20mM alpha-kg | 20mM pyruvate 10mM oaa |

| 20mM pyruvate 20mM fumarate 10mM oaa | 20mM alpha-kg 20mM fumarate 10mM oaa | 20mM pyruvate 20mM alpha-kg 10mM oaa | 20mM pyruvate 20mM alpha-kg 20mM fumarate |

| 20mM pyruvate 20mM alpha-kg 20mM fumarate 10mM oaa | 10mM pyruvate 10mM alpha-kg 10mM fumarate 5mM oaa |

Figure 2    Experimental design for block # 1 of the TCA cycle intermediates
interaction study

Malate Intermediates Interaction Experiment
Block 2

| Control | 5mM malate |
|---|---|

| 20mM alpha-kg<br>5mM malate | 20mM pyruvate<br>5mM malate | 20mM fumarate<br>5mM malate | 10mM oaa<br>5mM malate |
|---|---|---|---|

| 20mM pyruvate<br>20mM alpha-kg<br>20mM fumarate<br>10mM oaa<br>5mM malate | 10mM pyruvate<br>10mM alpha-kg<br>10mM fumarate<br>5mM oaa<br>2.5mM malate |
|---|---|

Figure 3        Experimental design for block # 2 of the TCA cycle intermediates
interaction study

Figure 4    Viable Cell Concentration for conditions, lines show average of duplicate shake flasks

Figure 5      Lactate accumulation in shake flask studies. Lines show average of duplicate conditions

Figure 6    Ammonium accumulation for all conditions tested. Lines show average of duplicate shake flasks for each condition tested

Figure 7    Ammonium accumulation for α-KG and oxaloacetic acid addition to culture. Lines show average of duplicate shake flasks for each condition tested

Figure 8    Cell-specific productivity of cultures treated with different levels of TCA intermediates. Error bars show value ranges

Figure 9      Viable cell concentration for all conditions tested in Block 1 and Block 2 of the DOE

Figure 10    Ammonia accumulation profiles for all conditions tested in Block 1 and Block 2 of the DOE.

Figure 11    Maximum ammonia accumulation for all conditions tested in Block 1 and Block 2 of the DOE

Figure 12 Final cell-specific productivity for all conditions tested in Block 1 and Block 2 of the DOE

Figure 13a

Figure 13b

Figure 13c

Figure 14a

Figure 14b

Figure 14c

Figure 15

Figure 16a

Figure 16b

Figure 16c

Figure 17a

Figure 17b

Figure 17c

Figure 18a

Figure 18b

Figure 18c

Figure 19

Figure 20

Figure 21

Figure 22

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- EP 307247 A
- US 4767704 A
- US 4657866 A
- US 4927762 A
- US 5122469 A
- US 4560655 A
- WO 9003430 A
- WO 8700195 A
- EP 0239400 A
- EP 054951 A
- WO 0029004 A
- US 20050043519 A

### Non-patent literature cited in the description

- Mammalian Cell Culture. Plenum Press, 1984
- **BARNES ; SATO.** *Cell,* 1980, vol. 22, 649
- **URLAUB ; CHASIN.** *Proc. Natl. Acad. Sci. USA,* 1980, vol. 77, 4216
- **MATHER.** *Biol. Reprod.,* 1980, vol. 23, 243-251
- **MATHER et al.** *Annals N.Y. Acad. Sci.,* 1982, vol. 383, 44-68
- **SAMBROOK et al.** Molecular Cloning: A Laboratory Manual. Cold Spring Harbor Laboratory Press, 1989, 7.3-7.57
- **SAMBROOK et al.** Molecular Cloning: A Laboratory Manual. Cold Spring Harbor Laboratory Press, 1989, 18.1-18.88
- **HAM ; WALLACE.** *Meth. Enz.,* 1979, vol. 58, 44
- **BARNES ; SATO.** *Anal. Biochem.,* 1980, vol. 102, 255
- American Type Culture Collection Catalogue of Cell Lines and Hybridomas. 1988, 346-349
- **HARLOW et al.** Antibodies A Laboratory Manual. Cold Spring Harbor Laboratory, 1988
- **QUEEN et al.** *Proc. Natl. Acad Sci USA,* 1989, vol. 86, 10029-10032
- **HODGSON et al.** *Bio/Technology,* 1991, vol. 9, 421
- **KABAT et al.** Sequences of Proteins of Immunological Interest. U.S. Department of Health and Human Services, National Institutes of Health, 1987
- **CHOTHIA et al.** Conformations of immunoglobulin hypervariable regions. *Nature,* 1989, vol. 342, 877-883
- *Mol. Immunol.,* 2006, vol. 44, 656-665